# EUROPEAN PATENT SPECIFICATION

(11) **EP 2 953 606 B1**
(45) Date of publication and mention of the grant of the patent: **13.09.2017**
(21) Application number: 14706113.9
(22) Date of filing: 05.02.2014
(51) Int. Cl.: A61K 8/31, A61Q 19/02, A61K 8/92

(54) **USE OF ORANGE ESSENTIAL OIL TO TREAT SKIN PIGMENTATION DISORDERS**
VERWENDUNG VON ÄTHERISCHEM ORANGENÖL ZUR BEHANDLUNG VON HAUTPIGMENTIERUNGSSTÖRUNGEN
UTILISATION D'HUILE ESSENTIELLE D'ORANGE POUR TRAITER DES TROUBLES DE LA PIGMENTATION DE LA PEAU

(30) Priority: 06.02.2013 FR 1351024; 11.02.2013 US 201361763128 P
(43) Date of publication of application: 16.12.2015
(73) Proprietor: L'Oréal, 75008 Paris (FR)
(72) Inventor: PEGEON, Agnès, F-92190 Meudon (FR); PELLETIER, Pascale, F-92160 Antony (FR); MARION, Catherine, F-92160 Antony (FR)
(74) Representative: Nony
(86) International application number: PCT/IB2014/058795
(87) International publication number: WO 2014/122584

(56) References cited:
- JP-A- 2010 106 000
- KR-A- 20090 086 051
- DATABASE WPI Week 201253 Thomson Scientific, London, GB; AN 2012-H00300 XP002714856, & CN 102 475 646 A (LIU S) 30 May 2012 (2012-05-30)
- DATABASE WPI Week 201350 Thomson Scientific, London, GB; AN 2013-H56048 XP002714858, & CN 102 846 520 A (QINGDAO KANGTAIXIN ENVIRONMENTAL PROTECT) 2 January 2013 (2013-01-02)
- DATABASE WPI Week 201182 Thomson Scientific, London, GB; AN 2011-P28063 XP002714859, & KR 2011 0121326 A (SHINANSEOMCHO) 7 November 2011 (2011-11-07)
- DATABASE WPI Week 200538 Thomson Scientific, London, GB; AN 2005-369329 XP002714860, & JP 2005 132793 A (UNIV KINKI) 26 May 2005 (2005-05-26)

## Description

The invention relates to the field of active cosmetic agents, and more particularly active agents for acting specifically on pigmentation of the skin and/or of the semi-mucous membranes.

Human skin consists of several compartments, three of which cover the whole of the body, namely a superficial compartment, which is the epidermis; the dermis; and a deep compartment, which is the hypodermis.

"Skin" refers to the whole of the skin on the body, including the scalp, the mucous and semi-mucous membranes, and the epidermal derivatives. "Epidermal derivatives" mean the body hair, eyelashes, head hair, and the nails. Contemplated more particularly in the present invention is the skin of the neckline, of the neck and of the face, and particularly the skin of the face.

The hypodermis is composed essentially of a type of cells specialized in the accumulation and storage of fats: the adipocytes.

The dermis is a connective tissue composed of collagen fibres and elastic fibres, and also of glycosaminoglycans, proteoglycans and fibroblasts. Its architecture results from the arrangement and the interactions between the constituents of the extracellular matrix and the fibroblasts which synthesize them and break them down.

The epidermis is composed primarily of keratinocytes, but also of other cells, more particularly melanocytes. These cells are located in a basal membrane which separates them from the dermis. The melanocytes are specialized dendritic cells, the function of which is to synthesize melanin.

Over time, and particularly as a result of chronological ageing and/or effects induced by light, the skin undergoes numerous modifications and degradations which are manifested, in the tissue, by disruption to the architecture of the epidermis, of the dermo-epidermal junction of the dermis, and also to the systems for blood irrigation and innervation, and in slowing-down or dysregulation of various cellular metabolisms, such as those involved in the equilibrium of the barrier function or involved in melanogenesis. At the cell level, ageing is therefore manifested in a change in physiology or metabolism in the principal types of cell, such as the fibroblasts of the dermis, the keratinocytes of the epidermis, and also the melanocytes. Thus, there is observed, in the melanocytes, an imbalance in the processes involved in the production of melanin. Furthermore, the distribution of this melanin at the surface of the skin appears to be detrimentally affected, thus resulting in a deterioration in the uniformity of the complexion, in the darkening thereof or in the appearance of pigmentary blemishes, or naevi, or depigmented regions, in particular in the regions exposed to light, in particular on the face, neckline, arms, hands and shoulders.

More particularly, senile pigmentary spots, also called lentigines, are represented macroscopically by benign pigmented spots of more or less dark brown colour, with edges that are defined but irregular. Highly variable in size, they may extend from a few millimetres to more than two centimetres.

These pigmentary disorders therefore give the skin a heterogeneity which, for obvious reasons, causes an aesthetic problem.

The removal or reduction in the presence of these disorders is based, generally speaking, on the application of depigmenting treatments.

For example, hydroquinone and derivatives thereof, more particularly its ethers such as hydroquinone monomethyl ether and monoethyl ether, exhibit a certain efficacy as depigmenting agents. These compounds, unfortunately, are not devoid of side effects, owing to their toxicity, and this makes them awkward or even dangerous to use. This toxicity arises in particular from the fact that these compounds intervene in fundamental mechanisms of melanogenesis, by killing cells, which therefore carries a risk of disturbing the biological environment of these cells and, consequently, forces the skin to evacuate them, producing toxins.

Consequently, the whole or partial replacement of hydroquinone by other active agents which have depigmentation properties but are devoid of the aforementioned drawbacks, thus being non-irritant, non-toxic to the skin, and stable in a composition, is of constant interest.

More specifically, therefore, a need exists for active cosmetic agents for improving the complexion of the skin, and particularly its uniformity, for promoting whiteness and/or lightening of the skin, and for preventing, reducing or treating pigmentation disorders of the skin and/or of the semi-mucous membranes such as the lips, more particularly the number, size, intensity and extent of pigmentary spots.

There is also a need for active cosmetic agents which have enhanced efficacy on the skin, particularly skin which is aged and/or has undergone repeated solar exposures.

There also remains a need for active agents that are capable of exerting a depigmenting or whitening action on an epidermis, in order to treat skin pigmentation disorders.

There also remains a need for active agents which do not have irritant and toxic side effects.

There remains more particularly a need for new essential oils which are capable of treating skin pigmentation disorders.

The aim of the present invention is to satisfy these needs.

Accordingly, according to a first aspect, the present invention relates to the cosmetic or dermatological use of at least one essential oil of sweet orange of the type *Citrus sinensis (L.) Osbeck* to prevent and/or treat skin pigmentation disorders.

According to another of its aspects, the present invention concerns the cosmetic or dermatological use of at least one essential oil of sweet orange of the type *Citrus sinensis (L.) Osbeck* to lighten the skin.

According to another of its aspects, the present invention concerns the cosmetic or dermatological use of at least one essential oil of sweet orange of the type *Citrus sinensis (L.) Osbeck* to prevent, reduce and/or treat an alteration in the complexion of the skin or the complexion of the semi-mucous membranes.

According to yet another of its aspects, the present invention relates to the cosmetic or dermatological use of at least one essential oil of sweet orange of the type *Citrus sinensis (L.) Osbeck* to stimulate, restore or regulate the metabolism of melanocytes of the skin and/or of the semi-mucous membranes.

The term "metabolism" is intended to denote, within the meaning of the present invention, the group of molecular and energy transformations which take place in a living cell and which contribute to its homeostasis, that is to say its maintenance of biological activity and of physiological balance.

In the sense of the present invention, "prevent" means at least partly lessening the risk of incidence of a given phenomenon, i.e., in the present invention, an adverse change in the metabolism of the melanocytes, particularly of aged melanocytes, or an adverse change in the complexion of the skin or the complexion of the semi-mucous membranes.

In the rest of the description, for purposes of simplification, the term "sweet orange" will be used indiscriminately to denote a sweet orange of the type *Citrus sinensis (L.)*

*Osbeck.* Unexpectedly, and as shown in the examples, it is the case that the inventors have observed that the sweet orange essential oil contemplated by the invention proves particularly advantageous in inducing a depigmenting effect on the melanocytes and the adjacent keratinocytes of the basal layer.

It is certainly the case that fruit extracts or essential oils have already been reported in whitening and/or depigmenting compositions.

Accordingly, JP2007063191 describes in particular an extract from immature fruits of *Citrus aurantium L*., or "Daïdaï", exhibiting an anti-tyrosinase activity with depigmenting properties. Documents RU2236862 and RU2222316, for their part, report compositions having a whitening effect and comprising a mixture of a number of essential oils, including essential oils of orange, grapefruit, mandarin and bitter oranges. Similarly, JP4703904 describes, in particular, a dentifrice product for tooth whitening, comprising essential oils of lemon, of orange and of grapefruit.
CN102475646 discloses a composition for whitening the skin containing sweet orange oil. The whitening effect is attributed to smoked plum and lemon.

CN102846520 discloses a facial cleanser composition which may whiten the skin, comprising a powder of orange peel. There is no indication of whitening or depigmentation activity of this component.

KR20110121326 discloses a soap for bleaching the skin comprising sweet orange. JP2005132793 discloses an agent for depigmenting the skin using an alcohol extract of unripe citrus, particularly Citrus reticulata.

JP2010106000 discloses that an extract of citrus leaves is effective against skin pigmentation. To the knowledge of the inventors, however, the particular properties of the essential oil of orange contemplated specifically according to the invention, namely sweet orange, as a whitening and/or depigmenting agent on keratin materials, more particularly the skin, have never been demonstrated.

Thus, essential oil of sweet orange is known in particular for its calmative and relaxing, disinfecting and deodorizing properties. It is stomachic, carminative, and is used as a tonic for digestion and the epidermis. It is also used externally, particularly in face creams for revitalizing the skin.

### Essential oils of sweet orange

In accordance with the definition given in International Standard ISO 9235 and adopted by the European Pharmacopoeia Commission, an essential oil is a product generally of complex composition, obtained from a botanically defined plant raw material, alternatively by steam entrainment, by dry distillation, or by an appropriate mechanical process without heating (cold pressing). The essential oil is generally separated from the aqueous phase via a physical process which does not result in any significant change in the composition.

An essential oil in accordance with the invention may therefore be prepared by the abovementioned techniques, and preferably by cold pressing.

In the context of the present invention, an essential oil may be prepared from any plant material obtained from *Citrus sinensis (L.) Osbeck* cultivated in vivo or obtained from in vitro culture.

An essential oil in accordance with the invention is preferably obtained from sweet orange peel.

The term "cultivating *in vivo*", or "culturing *in vivo*", is understood to mean any cultivation of conventional type, that is to say in soil in the open air or in a greenhouse, or alternatively without soil.

The term "cultivating *in vitro*", or "culturing *in vitro*" is understood to mean all the techniques known to those skilled in the art which make it possible to artificially obtain a plant or a plant part. The selection pressure imposed by the physicochemical conditions during the growth of plant cells *in vitro* makes it possible to obtain a standardized plant material that is available throughout the year, in contrast with plants cultivated *in vitro.*

According to one preferred embodiment, a sweet orange essential oil may be obtained by cold pressing from sweet orange peel.

The principle of the method is as follows: the peel is shredded and the contents of the secretory cavities which have been ruptured are recovered by a physical process. The conventional process consists in exerting an abrasive action over the entire surface of the fruit under a stream of water. After removal of the solid waste, the essential oil is separated from the aqueous phase by centrifuging. The majority of industrial plants in fact allow the simultaneous or sequential recovery of the fruit juices and of the essential oil.

The oil contains primarily monoterpene compounds. These monoterpenes are myrcene, alpha- and beta-pinenes, sabinene, and essentially limonene in D-limonene form. Thus the sweet orange essential oils advantageously possess a limonene content of more than 89% by total weight, more particularly more than 91%, and preferably more than 93%. Myrcene is present therein only at a level of 1.5% to 3.5% by total weight; alpha- and beta-pinenes and sabinene only as traces.

According to one preferred embodiment, an essential oil suitable for the invention has a limonene content, more particularly a D-limonene content, of more than 89%, preferably more than 93%, more particularly at from 89% to 97%, very particularly from 91% to 97%, preferably at from 93% to 96%.

The contents indicated above are given with respect to the total weight of the essential oil in question.

Apart from monoterpenes, the essential oils of the invention also comprise monoterpenols, sesquiterpenes, and terpene aldehydes in trace form. These include more particularly linalool, octanal, nonanal, decanal, neral, geraniol, geranial, valencene, and sinensal.

One such oil may be sold by the company Elixens (sweet orange essential oil - Ref. EVKO030D).

According to one preferred embodiment, the sweet orange essential oil is devoid of furocoumarin.

### Use of the sweet orange essential oils

As specified above, the sweet orange essential oils display particular depigmenting properties, more particularly aesthetic properties with regard to skin pigmentation disorders.

These skin pigmentation disorders are frequent and may manifest themselves in a variety of different forms. However, the skin pigmentation disorders to which the invention relates may be defined as involving the incidence of at least one skin region whose colour is darker or lighter than the average colour of the skin surface of the individual under examination. This region can be macroscopic or microscopic in size.

Skin to which the present invention relates may be elderly skin.

In the sense of the invention, the term "skin pigmentation disorders" refers to any event of incidence of a change in the colour, or the complexion, of all or part of the surface of the skin, giving rise to a local or general change in the complexion of the skin, and also hyperpigmentary, hypopigmentary or dyspigmentary disorders.

In the sense of the invention, the term "hyperpigmentary skin disorder" means any event of incidence of a skin region affected by excess pigmentation, by comparison with the average level of pigmentation of the skin surface of an individual.

It can relate, for example on dark skin types, to skin regions close to the joints, such as the interphalangeal regions, elbows, knees, nape of the neck or else the back of the hand or the heels at the rear of the talus.

The level of pigmentation of a skin surface can be measured using any appropriate type of colour-analysing instrument, for example with an appropriate spectrocolorimetry instrument, or else a reflectometer instrument, which are well known in the art.

The skin pigmentation disorders may be manifested by the presence of superficial skin blemishes of greater or lesser extent, with a colour darker than or lighter than the normal colour of the individual's skin surrounding said skin blemishes.

The skin pigmentation disorders include, in particular, melasma and lentigines, including senile lentigo and actinic lentigo.

Melasma (also known as chloasma) is most frequently encountered in pregnant women and in women taking anti-ovulatory medicaments. Melasma is also known as "mask of pregnancy". Melasma appears as a broad dark reticular macula with uneven edges which is found mainly on the cheeks, upper lip and forehead. Melasma is also encountered in men or women not suffering from detectable endocrinal imbalance, but exposure to the sun is necessary for its development.

Lentigines arise in the form of hyperpigmented skin blemishes which can appear at any age and are usually darker and more extensive than freckles. A lentigo is characterized in particular by an increase in the number of melanocytes in the basal layer.

Unaesthetic skin pigmentation disorders also encompass situations of hyperpigmentation subsequent to inflammation or to scarring. Post-inflammatory hyperpigmentation tends to be independent of the degree of inflammation, and dependent more on the nature of the trauma that gave rise to the inflammation.

The skin pigmentation disorders to which the invention relates also concern dyspigmentations resulting from metabolic dysfunction of the skin melanocytes, or else from an imbalance. Examples of dyspigmentations to which the invention relates include depigmentation due to burns or to surgery, post-acne spots, and cuts after shaving.

The skin pigmentation disorders to which the invention relates more particularly are, in particular, melasma, chloasma, lentigines, senile lentigo, post-inflammatory hyperpigmentations caused by an abrasion and/or a burn and/or a scar, hyperpigmentations of medicamentary origin, or any other pigmentary lesions.

The invention accordingly relates, in one of its aspects, to the use of a sweet orange essential oil, in which the skin pigmentation disorder is selected from melasma, chloasma, lentigines, senile lentigo, ephelides (freckles), pigmentary sequelae of acne, post-inflammatory pigmentation, meadow dermatitis, pigmentation linked to the poison ivy plant, benign dyschromias of the face, post-inflammatory hyperpigmentations caused by abrasion and/or burn and/or scar, hyperpigmentations of medicamentary origin, or any other pigmentary lesions.

The sweet orange essential oil according to the invention allows effective depigmentation and/or lightening of the skin of human beings. It is intended particularly for application to the skin of individuals having brownish pigmentation blemishes, age blemishes, or to the skin of individuals who wish to combat the appearance of a brownish colour originating from melanogenesis, for example following exposure to ultra-violet radiation. It may also allow a depigmentation and/or lightening of the body hair, eyelashes, head hair, and also the lips and/or nails.

The invention accordingly further provides a cosmetic process for depigmentation, lightening and/or whitening of the human skin, comprising the application to the skin of a composition as described above. The process is suitable particularly for eliminating brownish pigmentary blemishes and/or age blemishes, and/or for lightening browned skin.

The invention further provides for the cosmetic use of a sweet orange essential oil as a skin depigmenting and/or whitening agent, particularly for eliminating pigmentary blemishes, age blemishes, and/or as anti-browning agents.

The present invention can also be employed to prevent, reduce and/or treat the mask of pregnancy.

An active agent of the invention may be employed more particularly for lightening or whitening the skin.

The present invention also relates to aesthetic skin disorders affecting the complexion of the skin.

Thus, the invention may suitably be employed to prevent, reduce and/or treat a lack of uniformity in the complexion of the skin, indeed even to improve the complexion of the skin, in particular of elderly skin.

The invention advantageously allows the brightness of the skin complexion to be promoted and maintained, in relation more particularly to a uniform complexion.

The present invention relates to the entire surface of the skin of an individual.

Preferably, the present invention can be employed with regard to the skin regions more regularly exposed to the sun, to UV radiation or to external stress factors, such as pollution, irritation due to rubbing, or cigarette smoke. In particular, the present invention can advantageously be employed with regard to the skin of the hands, face or neckline.

### Composition of essential oils

The sweet orange essential oil may advantageously be formulated in a cosmetic or dermatological composition.

Preferably, a composition of the invention is a cosmetic composition.

According to one embodiment, a composition of this kind comprises essential oils according to the invention in an amount of from 0.00001% to 2.5% by weight with respect to the total weight of said composition, preferably from 0.0001% to 2.5%, preferably from 0.005% to 2%, more preferably from 0.01% to 1%, more preferably from 0.1% to 0.6% by weight, with respect to the total weight of the composition.

Of course, a composition comprising an amount of essential oils according to the invention of from 0.00001% to 2.5% may include all the sub-intervals in an individualized manner. In this respect, such a composition may comprise an amount of essential oils according to the invention of 0.1% ; 0.2% ; 0.3% ; 0.4% ; 0.5% ; 0.6% ; 0.7% ; 0.8% ; 0.9% ; 1%; 1.1% ; 1.2% ; 1.3% ; 1.4% ; 1.5% ; 1.6%; 1.7%; 1.8%; 1.9%; 2%; 2.1%; 2.2%; 2.3% ; 2.4% or 2.5%, by weight with respect to the total weight of said composition.

Thus, such a composition may comprise an amount of essential oils according to the invention of 0.1%; 0.2%; 0.3%; 0.4%; 0.5% or 0.6% by weight with respect to the total weight of said composition.

Thus, such a composition may comprise an amount of essential oils according to the invention of 0.1 to 0.6% by weight with respect to the total weight of said composition, which includes 0.1%; 0.2%; 0.3%; 0.4%; 0.5% and 0.6% by weight with respect to the total weight of said composition.

Thus, such a composition may comprise an amount of essential oils according to the invention varying from 0.1 to 0.6% by weight with respect to the total weight of said composition, which includes from 0.3 to 0.6% and from 0.4 to 0.6% by weight with respect to the total weight of said composition.

According to an exemplary embodiment, such a composition may comprise an amount of essential oils according to the invention of 0.5% by weight with respect to the total weight of said composition.

According to one particular embodiment, a sweet orange essential oil according to the invention may be present in a composition as a mixture with other essential oils, but distinct from the aforementioned varieties.

According to another embodiment, a sweet orange essential oil according to the invention may be present in a composition which does not comprise another type of essential oil that is distinct from the aforementioned varieties.

The sweet orange essential oil is preferably intended for topical administration, i.e. by surface application to the keratin material in question.

Accordingly, the compositions according to the invention may be in the form of products for caring for the skin or the semi-mucous membranes, such as a protecting, treating or care composition for the face, for the lips, for the hands, for the feet, for the anatomical folds or for the body (for example day creams, night cream, makeup remover cream, makeup base, anti-sun composition, body milk for protection or care, after-sun milk, a lotion, gel or mousse for care of the skin or the scalp, serum, powder, mask, artificial tanning composition, after-shave composition, hair composition, product for the axillary region, or product for hygiene and cleansing).

The essential oil therein is formulated in a physiologically acceptable medium.

For the purpose of the present invention, the term "physiologically acceptable medium" is intended to mean a medium that is suitable for the topical administration of a composition. A physiologically acceptable medium is preferably a cosmetically or dermatologically acceptable medium, that is to say a medium which is devoid of unpleasant odour or appearance and which is entirely compatible with the administration route under consideration.

When the composition is intended for topical administration, that is to say for administration by application at the surface of the keratinous substance under consideration, such a medium is considered to be physiologically acceptable when it does not cause stinging, tightness or redness unacceptable to the user.

The composition according to the invention may be present in any of the formulating forms normally used in the fields of cosmetology and dermatology.

It may be in the form, in particular, of an aqueous, aqueous-alcoholic or oily solution, optionally in gelled form, or an optionally two- or three-phase lotion-type dispersion, an oil-in-water or water-in-oil or multiple emulsion, an aqueous gel, a dispersion of oils in an aqueous phase, in particular by means of spherules, these spherules possibly being polymeric particles or, more preferably, ionic and/or nonionic lipid vesicles, or else in the form of a powder, a serum, a paste, a solid cake disintegrable by friction from an applicator or a soft or rigid stick or stylus, and meltable on the skin or the semi-mucous membranes. It can have a solid, pasty or more or less fluid liquid consistency.

With regard to the formulational form in question, the sweet orange essential oil may be formulated with the usual constituents.

As a non-limiting illustration of these customary components, mention may be made in particular of water, solvents, volatile or non-volatile oils, particularly as detailed hereinafter, waxes, pigments, fillers, surfactants, gellants, preservatives, colorants, antioxidants, UV screening agents and mixtures thereof.

Of course, a person skilled in the art will take care to choose the optional additional compound(s) and/or their amount so that the advantageous properties of the main active agent according to the invention are not, or not substantially, detrimentally affected by the envisaged addition and so that the properties of the compositions resulting therefrom are compatible with the administration route favoured.

A composition according to the invention may advantageously take the form of an emulsion, particularly an emulsion obtained by dispersing an aqueous phase in a fatty phase (W/O) or a fatty phase in an aqueous phase (O/W), with a liquid or semi-liquid consistency of the milk type, or with a soft, semi-solid or solid consistency of the cream of gel type, or else a multiple emulsion (W/O/W or O/W/O). These compositions are prepared according to the usual methods. The composition takes the form advantageously of an O/W (Oil in Water) emulsion.

Accordingly, a composition according to the invention may advantageously comprise from 0.1% to 99.9% by weight, and preferably from 30% to 95% by weight, of water, with respect to the total weight of said composition.

A composition according to the invention can also advantageously comprise at least one fatty phase which is liquid at ambient temperature and atmospheric pressure.

The amount of oily phase present in the compositions according to the invention can range, for example, from 0.01% to 50% by weight and preferably from 0.1% to 30% by weight, with respect to the total weight of the composition.

Mention may be made, as examples of oils which can be used in the composition according to the invention, of hydrocarbon oils of animal origin, hydrocarbon oils of vegetable origin, synthetic esters and ethers, in particular of fatty acids, linear or branched hydrocarbons of mineral or synthetic origin, fatty alcohols and silicone oils and their mixtures.

Other fatty substances which may be present in the oily phase are, for example, waxes, fatty alcohols and fatty acids comprising from 8 to 30 carbon atoms, such as stearic acid, lauric acid, palmitic acid and oleic acid. These fatty substances may be selected variously by a person skilled in the art so as to prepare a composition having the desired properties of, for example, consistency or texture.

Likewise, a composition according to the invention can additionally comprise at least one colorant selected, for example, from pigments, pearlescent agents, dyes, effect materials and their mixtures.

These colorants may be present in an amount of from 0.01% to 50% by weight with respect to the total weight of the composition, preferably from 0.01% to 30%.

A composition according to the invention may further comprise at least one filler, in particular in an amount of from 0.01% to 50% by weight, with respect to the total weight of the composition, preferably of from 0.01% to 30%. These fillers can be inorganic or organic and their choice comes within the competence of a person skilled in the art.

According to one of its aspects, the present invention is directed to a cosmetic process for lightening the skin, comprising at least one step of administering to an individual in need thereof essential oil of sweet orange, of the type *Citrus sinensis (L.) Osbeck,* in particular topically.

According to yet another of its aspects, the present invention relates to a cosmetic process for preventing, reducing and/or treating an alteration in the complexion of the skin, comprising at least one step of administering to an individual in need thereof essential oil of sweet orange, of the type *Citrus sinensis (L.) Osbeck,* in particular topically.

According to yet another of its aspects, the present invention relates to a cosmetic process for stimulating, restoring or regulating the metabolism of melanocytes, especially aged melanocytes of the skin or semi-mucous membranes, comprising at least one step of administering to an individual in need thereof essential oil of sweet orange, of the type *Citrus sinensis (L.) Osbeck,* in particular topically.

A cosmetic process according to the invention can be carried out daily, for example at the rate of a single administration per day or of an administration split up into two or three times per day, for example once in the morning and once in the evening.

### Packaging article

A packaging article of the invention will naturally be chosen by a person skilled in the art according to the presentation form of the composition to be packaged.

Thus, for liquid compositions, use may be made of containers consisting of a rigid envelope comprising means for dispensing the composition. These dispensing means may be a simple orifice, which is blocked by a removable cap, or a push button associated with a pump for expelling a part of the composition contained in the container. The compositions of the invention in liquid form may also be conditioned in containers of aerosol can type.

A composition of the invention in semi-liquid or pasty form may be advantageously conditioned in a jar, a tube of cream, or in a container with flexible or deformable walls and having an orifice that can be stoppered with a removable lid, the composition being expelled through the orifice by pressing on the walls, or a bottle equipped with a push button and a pump as indicated previously.

According to a particular embodiment, a conditioning article that is suitable for receiving a composition according to the invention may be made of glass, metal, alloy, coated papers such as wax-coated papers, for instance papers coated with beeswax, which especially have properties as a natural preserving agent.

Alternatively, a composition according to the invention may be stored under vacuum, in a hermetically sealed airtight compartment, like, for example, a brick-carton under vacuum, commonly used in the food sector.

The conditioning article may be at least partly made of plastics or other suitable polymeric materials.

According to a particular embodiment, the conditioning article can also be made using materials which make it possible to isolate the composition from any light sources.

The conditioning article can also be at least partly made of thermal insulation materials. By way of examples of materials of this type, mention may be made of fabrics, fabrics made of glass fibres coated with silicone, textiles based on ceramic fibres, cellulose fibres, polystyrene, styrofoam, and packaging films. A cold gel or liquid may also be used as thermal insulation material.

According to a particular embodiment, the conditioning article is disposable and opened just before its use by the consumer.

According to one embodiment, a composition of the invention formulated in solid form may be conditioned, for example, in a jar or a tube for a stick, for example a lipstick tube.

A packaging article of the invention may further comprise any means for applying a cosmetic composition that is commonly employed, such as a brush, tweezers or a pad.

In the description and in the examples which follow, unless otherwise indicated, the percentages are percentages by weight and the ranges of values given in the form "between ... and ..." include the upper and lower end points specified.

Throughout the description, including the claims, the expression "comprising a" should be understood as being synonymous with "comprising at least one", unless otherwise specified.

The examples and figures which follow are presented by way of illustration and without implied limitation of the invention. The percentages are expressed by weight of starting materials. The compounds, where appropriate, are cited with chemical names or with CTFA names (International Cosmetic Ingredient Dictionary and Handbook).

### EXAMPLES

### Example 1

### Composition for O/W emulsion of the invention.

This is a depigmenting cream which is applied topically one to two times daily.

| **Chemical name** | **Weight percentage (%)** |
|---|---|
| Stearate and PEG-100 Stearate - SIMULSOL™ 165, SEPPIC | 0.3 |
| Fatty acids (majority stearic acid) of plant origin - Stearine TP1200 pellets (DUB 50P) from Stearinerie DUBOIS | 0.6 |
| Cetyl alcohol- | 0.5 |
| Caprylic/capric (60/40) triglycerides - **MYRITOL 318®, COGNIS** | 3 |
| Propyl P-Hydroxybenzoate | 0.3 |
| Isocetyl Stearate | 5 |
| Glycerol | 7 |
| Methyl P-Hydroxybenzoate | 0.2 |
| Ethylenediaminetetraacetic acid, disodium salt, 2 H₂0 | 0.1 |
| Xanthan Gum - RHODICARE XC RHODIA | 0.15 |
| Sweet orange essential oil - EVKO030D from ELIXENS | 0.5 |
| Polyacrylamide and C₁₃₋₁₄ Isoparaffin and Laureth-7 - SEPIGEL 305™, SEPPIC | 1.8 |
| Fragrance | 0.09 |
| Deionized water | qs 100 |

The fatty phase, mixed with the emulsifier SIMULSOL™ 165 from SEPPIC, is heated to 70°C. The aqueous phase is heated to the same temperature.

The emulsion is formed by pouring the fatty phase into the aqueous phase, then adding the SEPIGEL at 60°C. After cooling to ambient temperature, the sweet orange essential oil is added.

### Example 2

### Evaluation of the depigmenting effect of sweet orange essential oil

### A. Equipment & Methods

### Maintained-survival skin model

Fragments of healthy human skin from 3 different donors of phototype III and IV (Fitzpatrick classification) were maintained under survival conditions. The products are topically applied daily. The skin fragments are then maintained under survival conditions for 10 days, then fixed in formaldehyde for histological analyses. The skin pigmentation was studied histologically under an optical microscope following visualization of the melanin pigment by Fontana-Masson staining of the melanocytes and the adjacent keratinocytes of the basal layer.

The score was evaluated: the cells of score 1 (low pigmentation), of score 2 (moderate pigmentation) or of score 3 (high pigmentation) were counted under each set of experimental conditions.

The statistical analysis is performed via the "reduced deviation" Student test or paired-samples test, with a risk of 5%.

### B. Results

A depigmenting effect is concluded if the percentage of pigmented cells (score 2 or 3) reduces or if the percentage of cells with low pigmentation (score 1) increases in the skins treated with the active agent, by comparison with skins from the same donor that have been treated with the placebo.

**Analysis of melanin pigmentation of the skin: % of epithelial cells of score 3**

| | **Skin 1** | **Skin 2** | **Skin 3** | **Average** |
|---|---|---|---|---|
| **Untreated control skin** | 55.4 | 86.4 | 60.7 | **67.5** |
| **Skin + ethanol - dodecane** | 58 | 73.1 | 54.7 | **62** |
| **Skin + sweet orange essential oil - ELIXENS at 0.00002%** | 49.7 | 62 | 35.17 | **49** |
| | | | | *p = 0.03 |

The decrease in the number of epithelial cells of score 3 is statistically significant with respect to the control (p < 0.05).

## Claims

1. Non-therapeutic, cosmetic use of at least one essential oil of sweet orange of the type *Citrus sinensis (L.) Osbeck* to prevent and/or treat skin pigmentation disorders.

2. Use according to Claim 1, in which the skin pigmentation disorder is selected from melasma, chloasma, lentigines, senile lentigo, ephelides, pigmentary sequelae of acne, post-inflammatory pigmentation, meadow dermatitis, pigmentation linked to the poison ivy plant, benign dyschromias of the face, post-inflammatory hyperpigmentations caused by an abrasion and/or a burn and/or a scar, hyperpigmentations of medicamentary origin, or any other pigmentary lesions.

3. Use according to either of the preceding claims, to lighten the skin.

4. Use according to any one of the preceding claims, to prevent, reduce and/or treat an alteration in the complexion of the skin.

5. Use according to any one of the preceding claims of at least one essential oil of sweet orange of the type *Citrus sinensis (L.) Osbeck* to stimulate, restore or regulate the metabolism of melanocytes of the skin and of the semi-mucous membranes.

6. Use according to any one of the preceding claims, in which the skin is elderly skin.

7. Use according to any one of the preceding claims, in which the essential oil is administered topically.

8. Use according to any one of the preceding claims, in the form of an O/W emulsion.

9. Use according to any one of the preceding claims, of an essential oil having a D-limonene content of more than 89% with respect to the total weight of said essential oil.

10. Use according to any one of the preceding claims, of an essential oil present in a composition in an amount of from 0.00001% to 2.5% with respect to the total weight of said composition, preferably from 0.0001% to 2.5%, preferably from 0.005% to 2%, more preferably from 0.01 % to 1%, more preferably from 0.1 % to 0.6%.

11. Non-therapeutic, cosmetic process for lightening the skin, comprising at least one step of administering to an individual in need thereof essential oil of sweet orange of the type *Citrus sinensis (L.) Osbeck.*

12. Non-therapeutic, cosmetic process for preventing, reducing and/or treating an alteration in the complexion of the skin, comprising at least one step of administering to an individual in need thereof essential oil of sweet orange of the type *Citrus sinensis (L.) Osbeck.*

13. Non-therapeutic, cosmetic process for stimulating, restoring or regulating the metabolism of melanocytes, especially aged melanocytes, of the skin or of the semi-mucous membranes, comprising at least one step of administering to an individual in need thereof essential oil of sweet orange of the type *Citrus sinensis (L.) Osbeck.*

## Patentansprüche

1. Nicht-therapeutische, kosmetische Verwendung von mindestens einem ätherischen Öl der Süßorange vom Typ *Citrus sinensis (L.) Osbeck* zum Vorbeugen und/oder Behandeln von Hautpigmentierungstörungen.

2. Verwendung gemäß Anspruch 1, wobei die Hautpigmentierungstörung aus Melasma, Chloasma, Leberflecken, Altersfleck, Sommersprossen, Pigmentfolgeerscheinungen von Akne, Pigmentierung nach Entzündung, Wiesengräserdermatitis, Pigmentierung im Zusammenhang mit der Giftefeu-Pflanze, benignen Dyschromien des Gesichts, Hyperpigmentierungen nach Entzündung, die durch eine Abschürfung und/oder eine Verbrennung und/oder eine Narbe verursacht wurden, Hyperpigmentierungen mit medikamentösem Ursprung oder jedweden anderen Pigmentläsionen ausgewählt ist.

3. Verwendung gemäß einem der vorhergehenden Ansprüche zum Aufhellen der Haut.

4. Verwendung gemäß einem der vorhergehenden Ansprüche zum Vorbeugen, Verringern und/oder Behandeln einer Veränderung der Hautfarbe.

5. Verwendung gemäß einem der vorhergehenden Ansprüche von mindestens einem ätherischen Öl der Süßorange vom Typ *Citrus sinensis (L.) Osbeck* zum Stimulieren, Wiederherstellen oder Regulieren des Metabolismus von Melanozyten der Haut und der Semischleimhäute.

6. Verwendung gemäß einem der vorhergehenden Ansprüche, wobei die Haut ältere Haut ist.

7. Verwendung gemäß einem der vorhergehenden Ansprüche, wobei das ätherische Öl topisch verabreicht wird.

8. Verwendung gemäß einem der vorhergehenden Ansprüche in der Form einer O/W-Emulsion.

9. Verwendung gemäß einem der vorhergehenden Ansprüche von einem ätherischen Öl mit einem Gehalt an D-Limonen von höher als 89 %, bezogen auf das Gesamtgewicht des ätherischen Öls.

10. Verwendung gemäß einem der vorhergehenden Ansprüche von einem ätherischen Öl, das in einer Zusammensetzung in einer Menge von 0,00001 % bis 2,5 %, bezogen auf das Gesamtgewicht der Zusammensetzung, bevorzugt 0,0001 % bis 2,5 %, bevorzugt 0,005 % bis 2 %, stärker bevorzugt 0,01 % bis 1 %, stärker bevorzugt 0,1 % bis 0,6 % vorhanden ist.

11. Nicht-therapeutisches, kosmetisches Verfahren zum Aufhellen der Haut, umfassend mindestens einen Schritt von Verabreichen an ein Individuum, welches dessen bedarf, eines ätherischen Öls der Süßorange vom Typ *Citrus sinensis (L.) Osbeck.*

12. Nicht-therapeutisches, kosmetisches Verfahren zum Vorbeugen, Verringern und/oder Behandeln einer Veränderung der Hautfarbe, umfassend mindestens einen Schritt von Verabreichen an ein Individuum, welches dessen bedarf, eines ätherischen Öls der Süßorange vom Typ *Citrus sinensis (L.) Osbeck.*

13. Nicht-therapeutisches, kosmetisches Verfahren zum Stimulieren, Wiederherstellen oder Regulieren des Metabolismus von Melanozyten, insbesondere gealterten Melanozyten, der Haut oder der Semischleimhäute, umfassend mindestens einen Schritt von Verabreichen an ein Individuum, welches dessen bedarf, eines ätherischen Öls der Süßorange vom Typ *Citrus sinensis (L.) Osbeck.*

## Revendications

1. Utilisation cosmétique non thérapeutique d'au moins une huile essentielle d'orange douce de type *Citrus sinensis (L.) Osbeck* pour prévenir et/ou traiter des troubles de la pigmentation cutanée.

2. Utilisation selon la revendication 1, dans laquelle le trouble de la pigmentation cutanée est choisi le mélasma, le chloasma, les lentigines, le lentigo sénile, les éphélides, les séquelles pigmentaires de l'acné, la pigmentation post-inflammatoire, la dermite des prés, la pigmentation liée à la plante de sumac vénéneux, les dyschromies bénignes du visage, les hyperpigmentations post-inflammatoires dues à une abrasion et/ou une brûlure et/ou une cicatrice, les hyperpigmentations d'origine médicamenteuse, ou toute autre lésion pigmentaire.

3. Utilisation selon l'une quelconque des revendications précédentes, pour éclaircir la peau.

4. Utilisation selon l'une quelconque des revendications précédentes, pour prévenir, réduire et/ou traiter une altération du teint de la peau.

5. Utilisation selon l'une quelconque des revendications précédentes d'au moins une huile essentielle d'orange douce de type *Citrus sinensis (L.) Osbeck* pour stimuler, restaurer ou réguler le métabolisme des mélanocytes de la peau et des semi-muqueuses.

6. Utilisation selon l'une quelconque des revendications précédentes, dans laquelle la peau est une peau âgée.

7. Utilisation selon l'une quelconque des revendications précédentes, dans laquelle l'huile essentielle est administrée par voie topique.

8. Utilisation selon l'une quelconque des revendications précédentes, sous la forme d'une émulsion H/E.

9. Utilisation selon l'une quelconque des revendications précédentes, d'une huile essentielle présentant une teneur en D-limonène supérieure à 89 % par rapport au poids total de ladite huile essentielle.

10. Utilisation selon l'une quelconque des revendications précédentes, d'une huile essentielle présente dans une composition en une teneur variant de 0,00001 % à 2,5 % par rapport au poids total de ladite composition, de préférence de 0,0001 % à 2,5 %, de préférence de 0,005 à 2 %, plus préférablement de 0,01 à 1 %, plus préférablement de 0,1 à 0,6 %.

11. Procédé cosmétique non thérapeutique pour éclaircir la peau comprenant au moins une étape d'administration à un individu en ayant besoin d'une huile essentielle d'orange douce de type *Citrus sinensis (L.) Osbeck.*

12. Procédé cosmétique non thérapeutique pour prévenir, réduire et/ou traiter une altération du teint de la peau comprenant au moins une étape d'administration à un individu en ayant besoin d'une huile essentielle d'orange douce de type *Citrus sinensis (L.) Osbeck.*

13. Procédé cosmétique non thérapeutique pour stimuler, restaurer ou réguler le métabolisme des mélanocytes, notamment des mélanocytes âgés, de la peau ou des semi-muqueuses, comprenant au moins une étape d'administration à un individu en ayant besoin d'une huile essentielle d'orange douce de type *Citrus sinensis (L.) Osbeck.*
